# EUROPEAN PATENT APPLICATION

(11) **EP 3 818 931 A1**
(43) Date of publication of application: **12.05.2021**
(21) Application number: 19830641.7
(22) Date of filing: 18.06.2019
(51) Int. Cl.: A61B 5/04, A61B 5/0404

(54) **ECG MEASUREMENT SYSTEM AND ECG TRANSMITTER**

(30) Priority: 06.07.2018 JP 2018129081
(71) Applicant: Nipro Corporation, Osaka-shi, Osaka 531-8510 (JP); Harada Electronics Industry Co., Ltd., Sapporo-shi, Hokkaido 063-0052 (JP)
(72) Inventor: NAKANO, Atsunori, Osaka-shi, Osaka 531-8510 (JP); KINOSHITA, Yoshiharu, Osaka-shi, Osaka 531-8510 (JP); HIEJIMA, Katsuhiro, Osaka-shi, Osaka 531-8510 (JP); UCHIDA, Tomoyuki, Osaka-shi, Osaka 531-8510 (JP); HARADA, Masahide, Sapporo-shi, Hokkaido 063-0052 (JP)
(74) Representative: Betten & Resch
(86) International application number: PCT/JP2019/024104
(87) International publication number: WO 2020/008864

(57) **Abstract**

1) the electrocardiographic transmitter 1 executes statistical processes for the electrocardiographic signal to calculate an average value HRav of the heart rate HR in a predetermined period of time. In a case where the average value HRav of the heart rate HR is abnormal, the electrocardiographic transmitter 1 continuously transmits the electrocardiogram data based on the electrocardiographic signal to the terminal 50. 2) The terminal 50 receives and transfers the electrocardiogram data as it is to the host device 70. 3) The host device 70 stores therein and supplies the transferred electrocardiogram data for a diagnosis by the medical worker. Thus, a state of the heart of a subject can be monitored for a long term.

## Description

### [TECHNICAL FIELD]

The present invention relates to an electrocardiographic measurement system capable of monitoring a state of the heart of a subject and an electrocardiographic transmitter used in the system.

### [BACKGROUND]

An electrocardiogram monitoring system has been proposed (for example, Patent Document 1), according to which a portable electrocardiograph having a communication function is attached to a subject having a heart disease, and electrocardiogram data are continuously transmitted in real time to a mobile terminal, and then a server receives the electrocardiogram data transmitted from the mobile terminal, and , in a case where irregular heartbeats occur in the subject, then the system acts to facilitate a diagnosis of a doctor by marking the time of occurrence of the irregular heartbeats on the server.

Further, an alarm system has been proposed (for example, Patent Document 2), according to which, in a case where arrhythmia occurs, a detection device attached to a subject detects that an abnormal waveform indicating arrhythmia is included in the electrocardiogram currently measured and transmits the detection signal toward a communication terminal located around the subject, thereby attracting attention of a person present around the subject to prompt the person to quickly cope with the arrhythmia.

### [PRIOR ART DOCUMENT]

### [PATENT DOCUMENT]

[Patent Document 1] JP 2018-19840 A
[Patent Document 2] JP 2017-209482 A

### [SUMMARY OF THE INVENTION]

### [PROBLEM TO BE SOLVED BY THE INVENTION]

In the system according to Patent Document 1, the portable electrocardiograph continuously transmits in real time the electrocardiogram data to the mobile terminal, consequently its power consumption tends to be large. The increase of the power consumption poses an obstacle to continuous measurement for a long term (such as, 1 month or longer) and, to realize it, it is unavoidable for a battery and the portable electrocardiograph incorporating therein the battery to each have an increased size and an increased weight. The increased size and the increased weight of the portable electrocardiograph continuously attached to the body for a long term lead to inconvenience of handling for the subject.

In the system according to Patent Document 2, the portable detection device detects in real time that the abnormal waveform indicating arrhythmia is included in the electrocardiogram currently measured and transmits the detection signal toward the communication terminal located around the user. The signal processing and the algorism for detecting the arrhythmia are therefore extremely complicated and the power consumption similarly tends to be large. The system does not have any functions of externally transmitting the electrocardiogram and a doctor cannot therefore diagnose the electrocardiogram in detail.

It is an object of the present invention to provide an electrocardiographic measurement system capable of monitoring a state of the heart of a subject for a long term.

It is another object of the present invention to provide an electrocardiographic transmitter whose power consumption is small and for which reduction of the size and the weight of each of a battery and the device is facilitated.

### [MEANS FOR SOLVING THE PROBLEM]

To achieve the above objects, the present invention is directed to an electrocardiographic measurement system including:
an attachable electrocardiographic transmitter that includes an electrocardiographic signal detection circuit configured to detect an electrocardiographic signal and a wireless communication circuit configured to wireless transmit the electrocardiographic signal, the transmitter capable of being attached to the body of a subject; and
a terminal configured to receive the electrocardiographic signal that is wireless transmitted from the electrocardiographic transmitter;
wherein the electrocardiographic transmitter executes a statistical process for the electrocardiographic signal to compute an amount of statistics and then transmits the amount of statistics to the terminal, and
the terminal determines whether or not the received amount of statistics is out of a predetermined range, and
the electrocardiographic transmitter transmits electrocardiogram data based on the electrocardiographic signal to the terminal in one case where the terminal determines that the amount of statistics is out of the predetermined range, and, on the other hand, does not transmit the electrocardiogram data to the terminal in the other case where the terminal determines that the amount of statistics is within the predetermined range.

Further, the present invention is directed to an electrocardiographic measurement system including:
an attachable electrocardiographic transmitter that includes an electrocardiographic signal detection circuit configured to detect an electrocardiographic signal and a wireless communication circuit configured to wireless transmit the electrocardiographic signal, the transmitter capable of being attached to the body of a subject; and
a terminal configured to receive the electrocardiographic signal that is wireless transmitted from the electrocardiographic transmitter;
wherein the electrocardiographic transmitter executes a statistical process for the electrocardiographic signal to compute an amount of statistics, and then determines whether or not the amount of statistics is out of a predetermined range and transmits electrocardiogram data based on the electrocardiographic signal to the terminal in one case where the electrocardiographic transmitter determines that the amount of statistics is out of the predetermined range, and, on the other hand, does not transmit the electrocardiogram data to the terminal in the other case where the electrocardiographic transmitter determines that the amount of statistics is within the predetermined range.

Furthermore, the present invention is directed to an electrocardiographic measurement system including:
an attachable electrocardiographic transmitter that includes an electrocardiographic signal detection circuit configured to detect an electrocardiographic signal and a wireless communication circuit configured to wireless transmit the electrocardiographic signal, the transmitter capable of being attached to the body of a subject; and
a terminal configured to receives the electrocardiographic signal that is wireless transmitted from the electrocardiographic transmitter;
wherein the electrocardiographic transmitter produces a synchronous signal synchronizing with the electrocardiographic signal and transmits the synchronous signal to the terminal, and
the terminal computes an amount of statistics of the electrocardiographic signal from the received amount of statistics and determines whether or not the amount of statistics is out of a predetermined range, and
the electrocardiographic transmitter transmits electrocardiogram data based on the electrocardiographic signal to the terminal in one case where the terminal determines that the amount of statistics is out of the predetermined range, and, on the other hand, does not transmit the electrocardiogram data to the terminal in the other case where the terminal determines that the amount of statistics is within the predetermined range.

According to the present invention, the statistical process is executed for the electrocardiographic signal to compute the amount of statistics, and the signal processing and the algorism can therefore be simplified. In a case where the amount of statistics is within the predetermined range (for example, in a normal range), the electrocardiogram data is not transmitted to the terminal and the electric power necessary for the data transmission can thereby be saved. The power consumption is therefore small, reduction of the size and the weight of each of a battery and the device is facilitated, and long-term monitoring of a state of the heart of a subject is thereby achieved.

It is preferred in the present invention that the system further includes a host device configured to receive and store therein the electrocardiogram data wireless transmitted from the terminal.

According to this aspect, the electrocardiogram data is stored in the host device, and a remote and detailed diagnosis of an electrocardiogram of a subject by a doctor is therefore enabled even in a case where the host device is located in a place remote from the electrocardiographic transmitter and the terminal.

It is preferred in the present invention that the amount of statistics is at least one parameter selected from the group consisting of average value, minimal value, maximal value, median value, most frequent value, dispersion, and standard deviation of heart rate in a predetermined period of time.

According to this aspect, these amounts of statistics can each be computed by simple signal processing and simple algorism, and the power consumption can therefore be reduced.

It is preferred in the present invention that the electrocardiographic transmitter may transmit a portion of the electrocardiogram data to the terminal at a predetermined time interval even in the case where the amount of statistics is within the predetermined range.

According to this aspect, a portion of the electrocardiogram data is transmitted to the terminal at the predetermined time interval even in the case where the amount of statistics is within the predetermined range, thereby the electrocardiogram of the subject can be diagnosed in a simplified manner.

Further, the present invention is directed to an attachable electrocardiographic transmitter including:
an electrocardiographic signal detection circuit configured to detect an electrocardiographic signal; and
a wireless communication circuit configured to wireless transmit the electrocardiographic signal to an external terminal;
the transmitter capable of being attached to the body of a subject,
wherein the electrocardiographic transmitter execute a statistical process for the electrocardiographic signal to compute a amount of statistics and then transmits the amount of statistics to the terminal, and transmits electrocardiogram data based on the electrocardiographic signal to the terminal in one case where the amount of statistics is out of a predetermined range, and, on the other hand, does not transmit the electrocardiogram data to the terminal in the other case where the amount of statistics is within the predetermined range.

Furthermore, the present invention is directed to an attachable electrocardiographic transmitter including:
an electrocardiographic signal detection circuit configured to detect an electrocardiographic signal; and
a wireless communication circuit configured to wireless transmit the electrocardiographic signal to an external terminal;
the transmitter capable of being attached to the body of a subject,
wherein the electrocardiographic transmitter executes a statistical process for the electrocardiographic signal to compute a amount of statistics, and determines whether or not the amount of statistics is out of a predetermined range, and then transmits electrocardiogram data based on the electrocardiographic signal to the terminal in one case where the amount of statistics is out of a predetermined range, and, on the other hand, does not transmit the electrocardiogram data to the terminal in the other case where the amount of statistics is within the predetermined range.

Furthermore, the present invention is directed to an attachable electrocardiographic transmitter including:
an electrocardiographic signal detection circuit configured to detect an electrocardiographic signal and
a wireless communication circuit configured to wireless transmit the electrocardiographic signal to an external terminal;
the transmitter capable of being attached to the body of a subject,
wherein the electrocardiographic transmitter produces a synchronous signal synchronizing with the electrocardiographic signal and transmits the synchronous signal to the terminal, and transmits electrocardiogram data based on the electrocardiographic signal to the terminal in one case where a amount of statistics of the electrocardiographic signal computed by the terminal from the synchronous signal is out of a predetermined range, and, on the other hand, does not transmit the electrocardiogram data to the terminal in the other case where the amount of statistics is within the predetermined range.

According to the present invention, a statistical process is executed for the electrocardiographic signal to compute the amount of statistics and the signal processing and the algorism can therefore be simplified. In a case where the amount of statistics is within the predetermined range (for example, in a normal range), the electrocardiogram data is not transmitted to the terminal and the electric power necessary for the data transmission can thereby be saved. The power consumption is therefore small, reduction of the size and the weight of each of a battery and the device can be facilitated, and long-term monitoring of the state of the heart of a subject is thereby achieved.

It is preferred in the present invention that the amount of statistics is at least one parameter selected from the group consisting of average value, minimal value, maximal value, median value, most frequent value, dispersion, and standard deviation of a heart rate in a predetermined period of time.

According to this aspect, these amounts of statistics can each be computed by simple signal processing and simple algorism, and the power consumption can therefore be reduced.

### [EFFECT OF THE INVENTION]

The present invention can provide an electrocardiographic measurement system capable of monitoring a state of the heart of a subject for a long term. The present invention can also provide an electrocardiographic transmitter whose power consumption is small and for which reduction of the size and the weight of each of a battery and the device is facilitated.

### [BRIEF DESCRIPTION OF THE DRAWINGS]

Fig. 1A is a perspective view showing one embodiment of an electrocardiographic transmitter according to the present invention, Fig. 1B is an explanatory view showing one example of the state where the electrocardiographic transmitter is attached to the chest of a subject, and Fig. 1C is an explanatory view showing one example of the state where an electrocardiogram is indicated on a display of a terminal.
Figs. 2A and 2B show one example of the outer appearance of the main body of the electrocardiographic transmitter, Fig. 2A is a plan view, and Fig. 2B is a bottom view showing the state where electrode pads are removed.
Fig. 3 is a block view showing one example of the electrical configuration of the electrocardiographic transmitter.
Fig. 4 is a configuration diagram showing one embodiment of an electrocardiographic measurement system according to the present invention.
Fig. 5 is a flowchart showing one example of operations of the electrocardiographic transmitter and the terminal.
Fig. 6 is a flowchart showing another example of the operations of the electrocardiographic transmitter and the terminal.
Fig. 7 is a flowchart showing further another example of the operations of the electrocardiographic transmitter and the terminal.
Fig. 8 is a flowchart showing further another example of the operations of the electrocardiographic transmitter and the terminal.
Fig. 9A is a graph showing one example of an electrocardiogram waveform, and Fig. 9B is a graph showing one example of a synchronous pulse of the electrocardiogram waveform.
Fig. 10 is a flowchart showing further another example of the operations of the electrocardiographic transmitter and the terminal.
Fig. 11 is a flowchart showing one example of operations of a heart rate meter and the terminal.

### [EMBODIMENT FOR CARRYING OUT THE INVENTION]

Hereinafter, embodiments of the present invention will be specifically described with reference to the accompanying drawings.

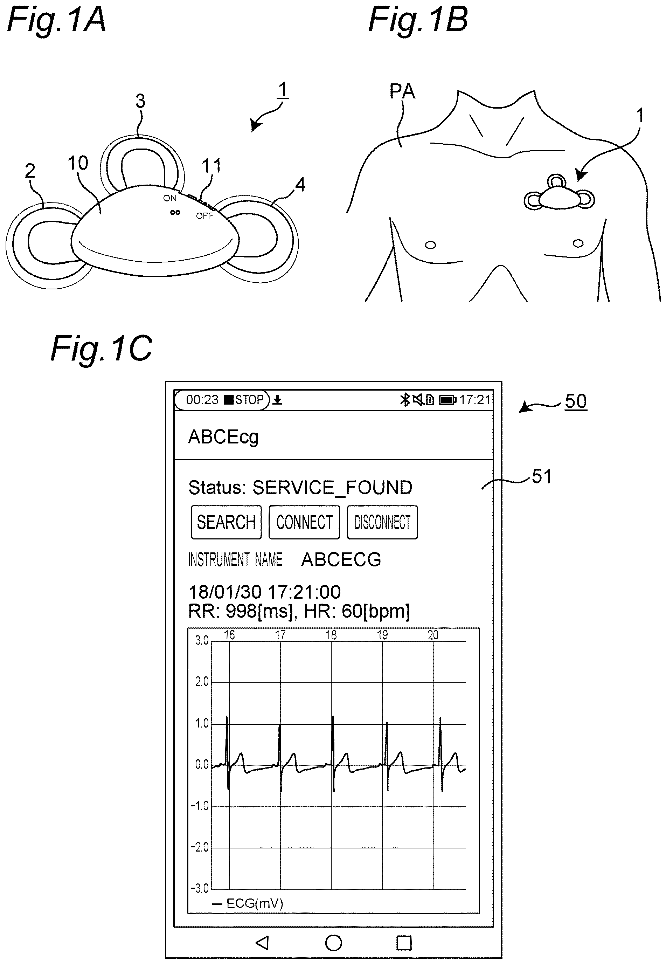

Fig. 1A is a perspective view showing one embodiment of an electrocardiographic transmitter 1 according to the present invention. Fig. 1B is an explanatory view showing one example of the state where the electrocardiographic transmitter 1 is attached to the chest of a subject PA. Fig. 1C is an explanatory view showing one example of the state where an electrocardiogram is indicated on a display 51 of a terminal 50.

A power switch 11 is disposed on a main body 10 of the electrocardiographic transmitter 1, and three electrode pads 2, 3, 4 can be attached to the back face of the main body 10. The electrocardiographic transmitter 1 as above is attached such that the electrode pads 2, 3, 4 are electrically connected to the chest of the subject PA, and thereby measuring an electrocardiographic signal. The shape of the outer appearance of the electrocardiographic transmitter 1 is not limited at all whenever the electrocardiographic transmitter 1 can measure the electrocardiographic signal of the subject PA and, for example, the electrode pads 2, 3, 4 may each be an integrated-type one that cannot be attached and detached from the main body 10, and the number of the pads may be two, three or more. The electrocardiographic transmitter 1 is wireless connected to the terminal 50 through a wireless communication standard that assumes a relatively short distance, such as Bluetooth (registered trademark), near field communication (NFC), personal area network (PAN), wireless local area network (LAN), or wireless fidelity (WiFi).

The terminal 50 includes, for example, a smartphone, a hand-held computer, a tablet PC, or a desktop PC and can have various types of software installed therein. Of these, dedicated software specialized in the electrocardiographic measurement system according to the present invention is installed into the terminal 50, the terminal 50 thereby can receive an electrocardiographic signal that is wireless transmitted from the electrocardiographic transmitter 1, and execute signal processing for the electrocardiographic signal, and store in a memory an electrocardiogram and various types of electrocardiographic parameter (such as, respiratory rate RR and heart rate HR) of the subject PA, and indicate them on the display 51, and transfer them to an external host device (not shown) . The terminal 50 is wireless connected to the host device through a wireless communication standard that assumes a relatively long distance, such as wireless local area network (LAN), wireless fidelity (WiFi), 3G, 4G (LTE), 5G, or the Internet.

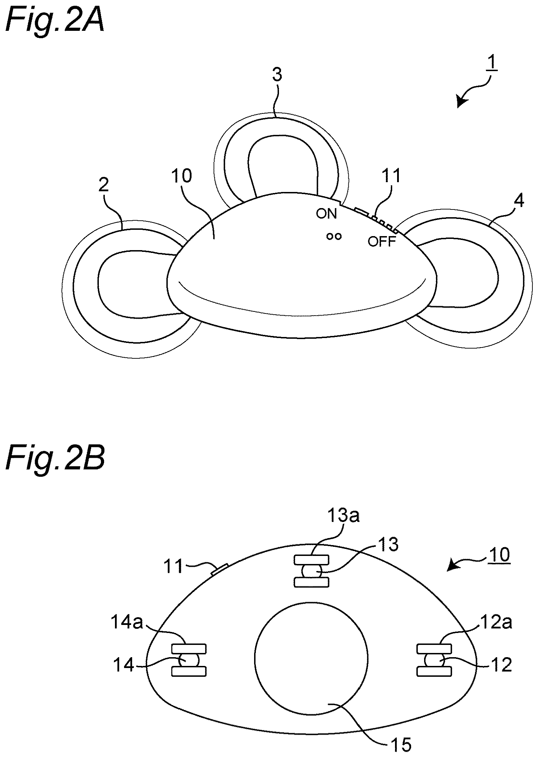

Figs. 2A and 2B show one example of the outer appearance of the main body 10 of the electrocardiographic transmitter 1, Fig. 2A is a plan view, and Fig. 2B is a bottom view showing the state where the electrode pads are removed.

The main body 10 of the electrocardiographic transmitter 1 has a rounded-corner triangular shape as a whole, and the sliding power switch 11 is disposed on a lateral side. As shown in Fig. 2B, terminals 12, 13, 14 respectively connected electrically to the electrode pads 2, 3, 4 are disposed on the bottom face, and pairs of fasteners 12a, 13a, 14a each respectively connected mechanically to the electrode pads 2, 3, 4 are disposed respectively in the vicinities of the terminals 12, 13, 14. A battery cover 15 to accommodate therein a battery (such as, button battery) is disposed at the center of the bottom face.

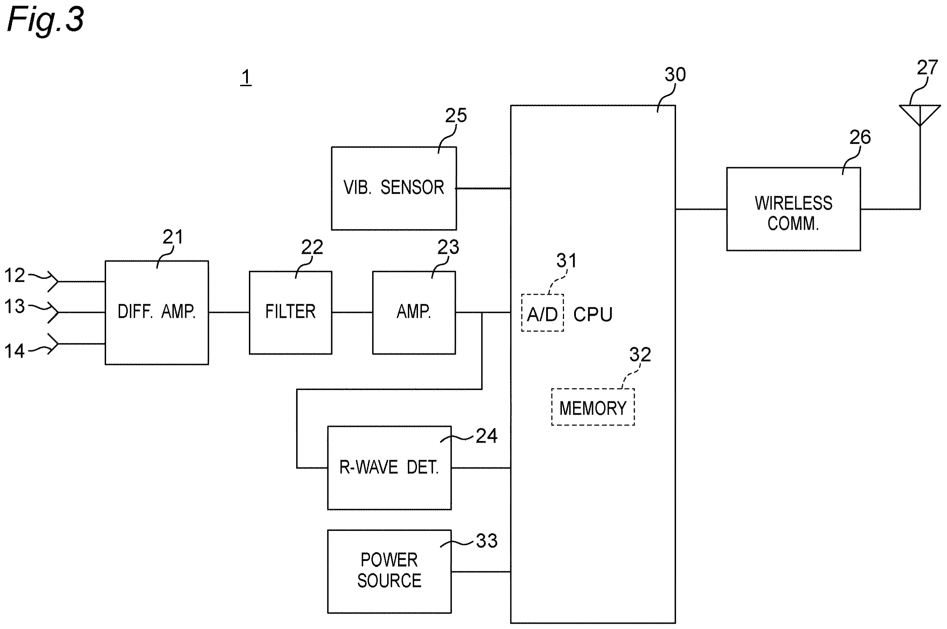

Fig. 3 is a block view showing one example of the electrical configuration of the electrocardiographic transmitter 1. The electrocardiographic transmitter 1 includes a differential amplifier 21 connected to the terminals 12, 13, 14, a filter 22, an amplifier 23, and an R-wave detection circuit 24 as an electrocardiographic signal detection circuit, and further includes a vibration sensor 25, a central processing unit (CPU) 30 incorporating therein an analog to digital (A/D) converter 31 and a memory 32, a wireless communication circuit 26, an antenna 27, a power source circuit 33, and the like.

The differential amplifier 21 has a function of detecting an electrocardiographic signal by amplifying the difference between a potential generated at the electrode pad 2 and a potential generated at the electrode pad 4 with a potential generated at the electrode pad 3 positioned at the center being a ground potential. In-phase components to be a noise can thereby be suppressed and reverse-phase components to be the signal can be amplified.

The filter 22 includes a high-pass filter that suppresses low-frequency noises such as a drift noise, a low-pass filter that suppresses high-frequency noises such as an electromyogram signal, a notch filter that suppresses specific frequency components such as a commercial power supply noise, and the like.

The amplifier 23 has a function of matching the electrocardiographic signal with the input range of the A/D converter 31 by amplifying the electrocardiographic signal. The A/D converter 31 converts the electrocardiographic signal into a digital signal, and the digital signal is stored in the memory 32.

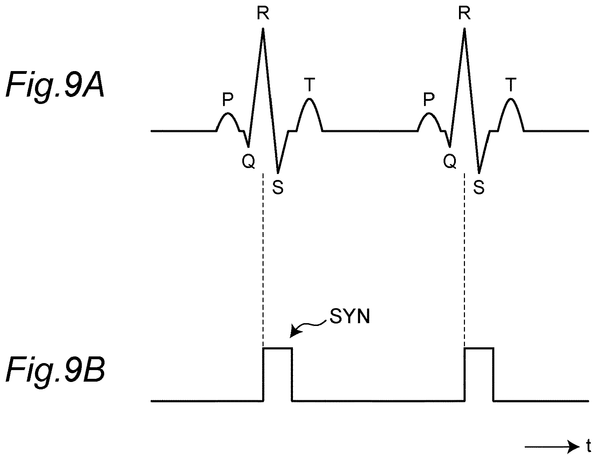

The R-wave detection circuit 24 detects a position of the R-wave that has the steepest peak of the electrocardiogram waveform (Fig. 9A) to produce a synchronous pulse SYN thereof (Fig. 9B) and supply it to the CPU 30. The CPU 30 can calculate various electrocardiographic parameters, such as RR (millisecond: ms) that is the peak interval of the R-wave and heart rate HR (beats/min: bpm) per unit time, based on the detected position of the R-wave. The CPU 30 can also wireless transmit the synchronous pulse SYN synchronizing with the R-wave, through the wireless communication circuit 26 to the terminal 50.

The vibration sensor 25 includes a three-axis acceleration sensor, which can detect a respiratory rate, a number of walking steps, an activity level, and the like of the subject PA and supply them to the CPU 30 through the A/D converter 31.

The CPU 30 operates in accordance with a predetermined program, and has a function of controlling the signal processing for the electrocardiographic signal and the like, and the operation of the overall device.

The wireless communication circuit 26 has functions of modulating a digital signal such as the electrocardiographic signal and a communication command of the CPU 30 each into a high-frequency signal and demodulating the high-frequency signal received from the terminal 50 into a digital signal, in accordance with the above wireless communication standard, such as Bluetooth (registered trademark).

The antenna 27 has functions of transmitting the high-frequency signals from the wireless communication circuit 26 and receiving the high-frequency signals from the terminal 50.

The power source circuit 33 includes the power switch 11, the battery, and the like.

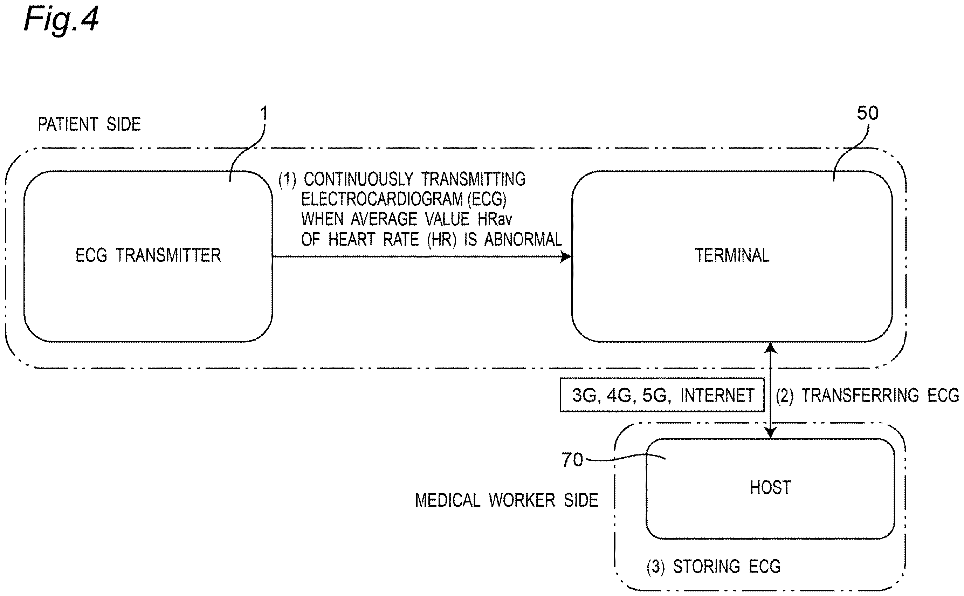

Fig. 4 is a configuration diagram showing one embodiment of the electrocardiographic measurement system according to the present invention. The electrocardiographic measurement system includes the above-mentioned electrocardiographic transmitter 1, the terminal 50 that wireless communicates with the electrocardiographic transmitter 1, and a host device 70 that wireless communicates with the terminal 50. The electrocardiographic transmitter 1 and the terminal 50 are located close to a patient and, on the other hand, the host 70 is usually located close to a medical worker standing by in a place remote from the patient.

With reference to Fig. 4, an operation of the overall system will be described below. First, 1) the electrocardiographic transmitter 1 executes statistical processes for the electrocardiographic signal to calculate an average value HRav of the heart rate HR in a predetermined period of time. In a case where the average value HRav of the heart rate HR is abnormal, the electrocardiographic transmitter 1 continuously transmits the electrocardiogram data based on the electrocardiographic signal to the terminal 50. 2) The terminal 50 receives the electrocardiogram data and transfers the electrocardiogram data as it is to the host device 70. 3) The host device 70 stores therein the transferred electrocardiogram data and supplies the electrocardiogram data for a diagnosis by the medical worker.

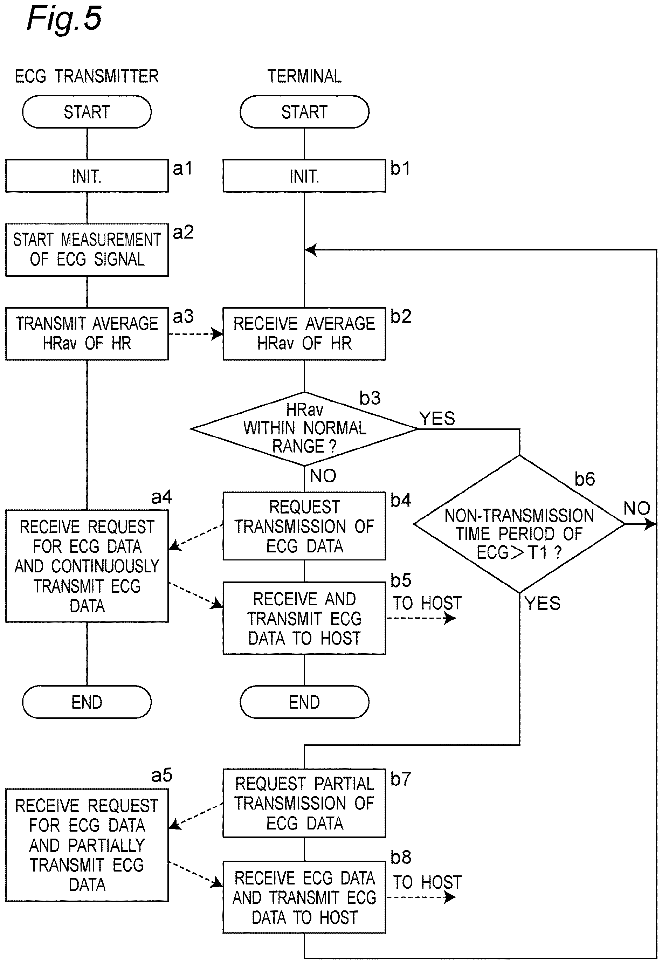

Fig. 5 is a flowchart showing one example of operations of the electrocardiographic transmitter 1 and the terminal 50. This example describes the case where the terminal 50 determines normality or abnormality of the average value HRav. When the electrocardiographic transmitter 1 is attached to the chest of the subject PA and the power switch 11 is turned on, initialization is executed at step a1 to make initial settings for, for example, the interval to calculate the average value HRav of the heart rate HR. Meanwhile, when the dedicated software installed in the terminal 50 is started up, initialization is executed at step b1 to make initial settings for, for example, the upper limit and the lower limit of a normal range of the average value HRav of the heart rate HR, a threshold value of a non-transmission time period of the electrocardiogram data, and the data amount of partial transmission of the electrocardiogram data, etc. Of these initially set values, at least the upper limit and the lower limit of the normal range of the average value HRav of the heart rate HR and the threshold value of the non-transmission time period of the electrocardiogram data may optionally be set by a person such as the subject PA or a medical worker. With Bluetooth (registered trademark), establishment of the communication between the electrocardiographic transmitter 1 and the terminal 50 is executed when the initialization and the data transmission are executed (the same is applied to other flowcharts).

The electrocardiographic transmitter 1 next starts measurement of the electrocardiographic signal at step a2, and then calculates the average value HRav of the heart rate HR in a predetermined period of time (such as, 1 minute) and transmits the average value HRav to the terminal 50 at step a3.

The terminal 50 receives the average value HRav of the heart rate HR transmitted from the electrocardiographic transmitter 1 at step b2, and determines whether or not the average value HRav is within the normal range (such as, 30 to 160 bpm) at step b3. When the terminal 50 determines that the average value HRav is within the normal range, the operation returns to step b2 through step b6 (whose details will be described later). In this manner, it can be determined that the subject PA is in a lull in the case where the average value HRav is normal. Thus, the electric power necessary for the data transmission can therefore be saved by omitting transmission of the electrocardiogram data having a huge data amount to the terminal 50.

On the other hand, in the case where the average value HRav is out of the normal range at step b3, it can be determined that the subject PA is in a serious condition, and the operation therefore moves to step b4 and the terminal 50 requests the electrocardiographic transmitter 1 to transmit the electrocardiogram data. The electrocardiographic transmitter 1 receives a request order for the electrocardiogram data and thereafter continuously transmits the electrocardiogram data based on the electrocardiographic signal to the terminal 50, at step a4. The terminal 50 receives the continuously transmitted electrocardiogram data and transfers the electrocardiogram data as it is to the host device 70, at step b5. In this manner, in the case where the average value HRav is abnormal, all of the electrocardiogram data of the subject PA can be supplied to the medical worker.

Next, step b6 will be described. In this example, a portion of the electrocardiogram data is transmitted to the terminal 50 even when the average value HRav is within the normal range. Specifically, the terminal 50 determines whether a non-transmission time period of the electrocardiogram data exceeds a predetermined period of time T1 (such as, 1 hour), at step b6. In the case where the non-transmission time period of the electrocardiogram data exceeds the period of time T1, the operation moves to step b7 and the terminal 50 requests the electrocardiographic transmitter 1 to transmit a portion of the electrocardiogram data (such as, a portion corresponding to 10 heartbeats). The electrocardiographic transmitter 1 receives a request order for the electrocardiogram data and thereafter transmits only the portion of the electrocardiogram data to the terminal 50, at step a5. The terminal 50 receives the portion of the electrocardiogram data and transfers this portion as it is to the host device 70 at step b8, and the operation returns to step b2. In this manner, the medical worker can diagnose the electrocardiogram of the subject PA in a simplified manner.

The above-mentioned example describes the case where the electrocardiographic transmitter 1 calculates the average value HRav of the heart rate HR. The electrocardiographic transmitter 1 can also transmit biomedical signal information such as the number of the R-waves measured by the electrocardiographic transmitter 1 to the terminal 50 and the software installed in the terminal 50 can also calculate the average value HRav of the heart rate HR using the biomedical signal information received by the terminal 50.

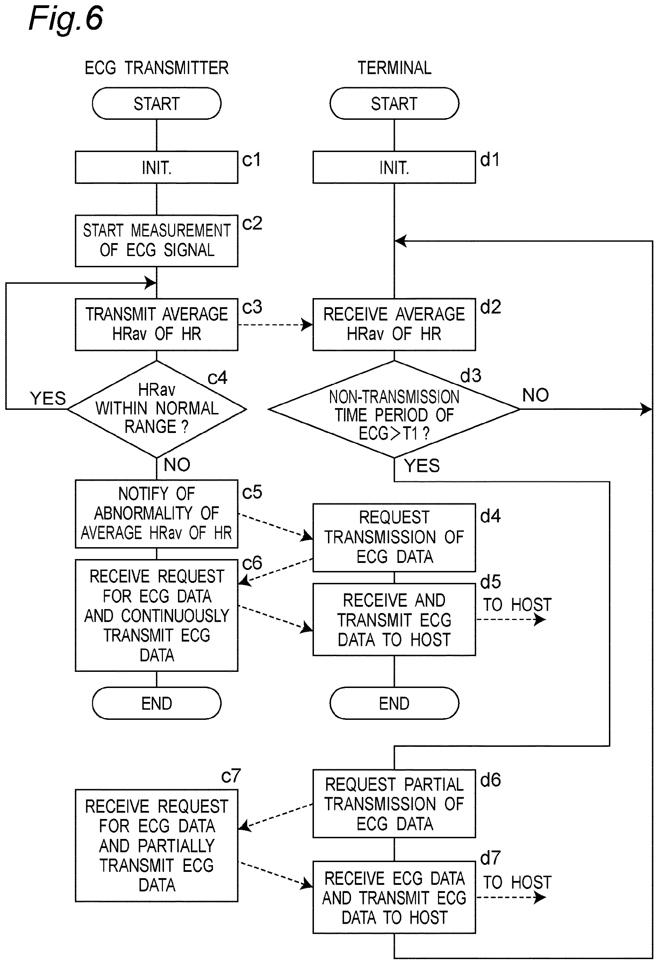

Fig. 6 is a flowchart showing another example of the operations of the electrocardiographic transmitter 1 and the terminal 50. This example describes the case where the electrocardiographic transmitter 1 determines normality or abnormality of the average value HRav. When the electrocardiographic transmitter 1 is attached to the chest of the subject PA and the power switch 11 is turned on, initialization is executed at step c1 to make initial settings for, for example, the interval to calculate the average value HRav of the heart rate HR, and the upper limit and the lower limit of the normal range of the average value HRav of the heart rate HR. Meanwhile, when the dedicated software installed in the terminal 50 is started up, initialization is executed at step c1 to make initial settings for, for example, a threshold value of a non-transmission time period of the electrocardiogram data, and the data amount of partial transmission of the electrocardiogram data, etc. The normal range of the average value HRav of the heart rate HR can also be input at the terminal 50, which transmits it to the electrocardiographic transmitter 1 and thereby the initial setting can be made.

The electrocardiographic transmitter 1 next starts measurement of the electrocardiographic signal at step c2, and then calculates the average value HRav of the heart rate HR in a predetermined period of time (such as, 1 minute) and transmits the average value HRav to the terminal 50 at step c3. The terminal 50 receives the average value HRav of the heart rate HR transmitted from the electrocardiographic transmitter 1, at step d2.

Subsequently, the electrocardiographic transmitter 1 determines at step c4 whether or not the average value HRav is within the normal range (such as, 30 to 160 bpm). When the average value HRav is within the normal range, the operation returns to step c3. In this manner, it can be determined that the subject PA is in a lull in the case where the average value HRav is normal. Thus, the electric power necessary for the data transmission can therefore be saved by omitting transmission of the electrocardiogram data having a huge data amount to the terminal 50.

On the other hand, in the case where the average value HRav is out of the normal range at step c4, it can be determined that the subject PA is in a serious condition, and the operation therefore moves to step c5 and the electrocardiographic transmitter 1 notifies the terminal 50 of the fact that the average value HRav of the heart rate HR is abnormal. The terminal 50 requests the electrocardiographic transmitter 1 to transmit the electrocardiogram data at step d4. The electrocardiographic transmitter 1 receives a request order for the electrocardiogram data and thereafter continuously transmits the electrocardiogram data based on the electrocardiographic signal to the terminal 50 at step c6. The terminal 50 receives the continuously transmitted electrocardiogram data and transfers the electrocardiogram data as it is to the host device 70 at step d5. In this manner, in the case where the average value HRav is abnormal, all of the electrocardiogram data of the subject PA can be supplied to the medical worker.

Next, step d3 will be described. In this example, a portion of the electrocardiogram data is transmitted to the terminal 50 even when the average value HRav is within the normal range. Specifically, the terminal 50 determines whether a non-transmission time period of the electrocardiogram data exceeds a predetermined period of time T1 (such as, 1 hour) at step d3. In the case where the non-transmission time period of the electrocardiogram data exceeds the period of time T1, the operation moves to step d6 and the terminal 50 requests the electrocardiographic transmitter 1 to transmit a portion of the electrocardiogram data (such as, a portion corresponding to 10 heartbeats). The electrocardiographic transmitter 1 receives a request order for the electrocardiogram data and thereafter transmits only the portion of the electrocardiogram data to the terminal 50, at step c7. The terminal 50 receives the portion of the electrocardiogram data and transfers this portion as it is to the host device 70 at step d7, and the operation returns to step d2. In this manner, the medical worker can diagnose the electrocardiogram of the subject PA in a simplified manner.

The above-mentioned example describes the case where the electrocardiographic transmitter 1 calculates the average value HRav of the heart rate HR. The electrocardiographic transmitter 1 can also transmit biomedical signal information such as the number of the R-waves measured by the electrocardiographic transmitter to the terminal 50 and the software installed in the terminal 50 can also calculate the average value HRav of the heart rate HR using the biomedical signal information received by the terminal 50.

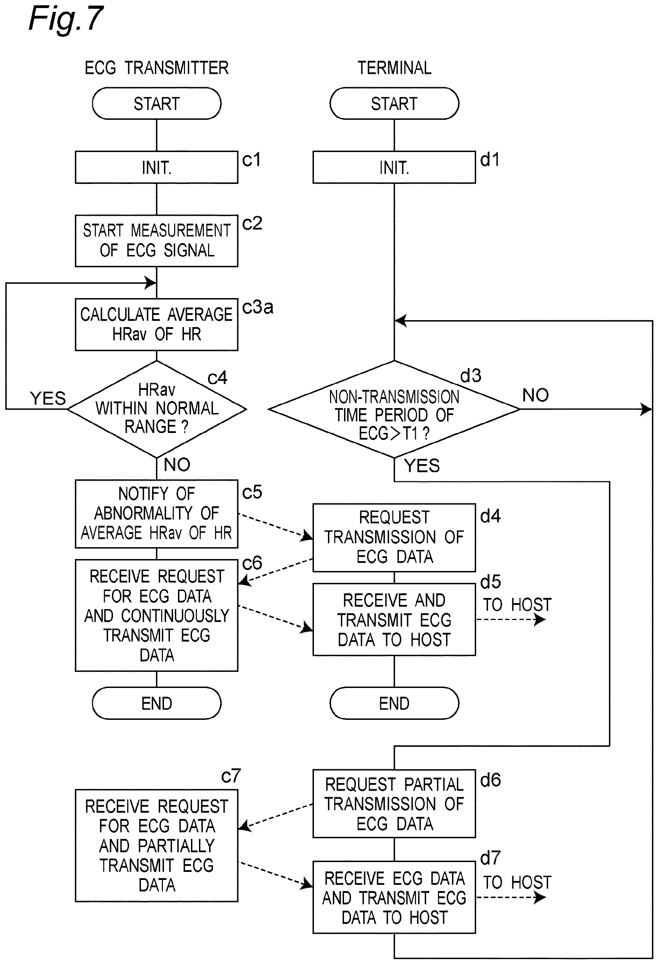

Fig. 7 is a flowchart showing further another example of the operations of the electrocardiographic transmitter 1 and the terminal 50. In this flowchart, step d2 in the flowchart shown in Fig. 6 is omitted and step c3a is executed instead of step c3, for calculating the average value HRav of the heart rate HR in a predetermined period of time (such as, 1 minute) without data transmission. The electrocardiographic transmitter 1 executes threshold determination for the average value HRav at subsequent step c4, while the electrocardiographic transmitter 1 transmits nothing to the terminal 50 as long as the average value HRav is normal. However, the electrocardiographic transmitter 1 executes the partial transmission of the electrocardiogram data (step c7). The frequency of the data communication is thereby reduced, and reduction of the power consumption and extension of the battery life can therefore be facilitated.

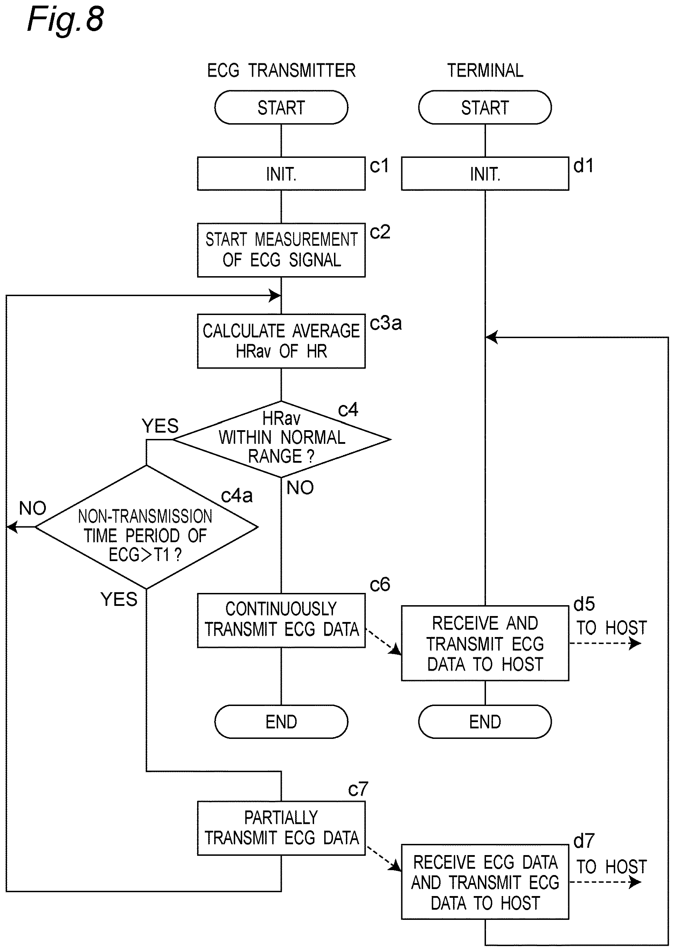

Fig. 8 is a flowchart showing further another example of the operations of the electrocardiographic transmitter 1 and the terminal 50. This example describes the case where the electrocardiographic transmitter 1 determines normality or abnormality of the average value HRav and also determines the non-transmission time period of the electrocardiogram data. When the electrocardiographic transmitter 1 is attached to the chest of the subject PA and the power switch 11 is turned on, initialization is executed at step c1 to make initial settings for, for example, the interval to calculate the average value HRav of the heart rate HR, and the upper limit and the lower limit of the normal range of the average value HRav of the heart rate HR. Meanwhile, when the dedicated software installed in the terminal 50 is started up, initialization is executed at step c1 to make initial settings for, for example, the threshold value for a non-transmission time period of the electrocardiogram data, and the data amount of the partial transmission of the electrocardiogram data, etc. The normal range of the average value HRav of the heart rate HR can also be input at the terminal 50, which transmits it to the electrocardiographic transmitter 1 and thereby the initial setting can also be made.

The electrocardiographic transmitter 1 next starts measurement of the electrocardiographic signal at step c2, and then calculates the average value HRav of the heart rate HR in a predetermined period of time (such as, 1 minute) without data transmission at step c3a.

Subsequently, the electrocardiographic transmitter 1 determines at step c4 whether or not the average value HRav is within the normal range (such as, 30 to 160 bpm). When the average value HRav is within the normal range, the operation returns through step c4a (whose details will be described later) to step c3a. In this manner, it can be determined that the subject PA is in a lull in the case where the electrocardiographic transmitter 1 determines that the average value HRav is normal. Thus, the electric power necessary for the data transmission can therefore be saved by omitting transmission of the electrocardiogram data having a huge data amount to the terminal 50.

On the other hand, in the case where the average value HRav is out of the normal range at step c4, it can be determined that the subject PA is in a serious condition, and the operation therefore moves to step c6 and the electrocardiographic transmitter 1 continuously transmits the electrocardiogram data based on the electrocardiographic signal to the terminal 50. The terminal 50 receives the continuously transmitted electrocardiogram data and transfers the electrocardiogram data as it is to the host device 70 at step d5. In this manner, in the case where the average value HRav is abnormal, all of the electrocardiogram data of the subject PA can be supplied to the medical worker.

Next, step c4a will be described. In this example, a portion of the electrocardiogram data is transmitted to the terminal 50 even when the average value HRav is within the normal range. Specifically, the electrocardiographic transmitter 1 determines whether a non-transmission time period of the electrocardiogram data exceeds a predetermined period of time T1 (such as, 1 hour) at step c4a. In the case where the electrocardiographic transmitter 1 determines that the non-transmission time period of the electrocardiogram data exceeds the period of time T1, the operation moves to step c7 and the electrocardiographic transmitter 1 transmits a portion of the electrocardiogram data (such as, a portion corresponding to 10 heartbeats) to the terminal 50. The terminal 50 receives the portion of the electrocardiogram data and transfers the portion as it is to the host device 70 at step d7, and thereafter the operation returns to the step following step d1. In this manner, the medical worker can diagnose the electrocardiogram of the subject PA in a simplified manner.

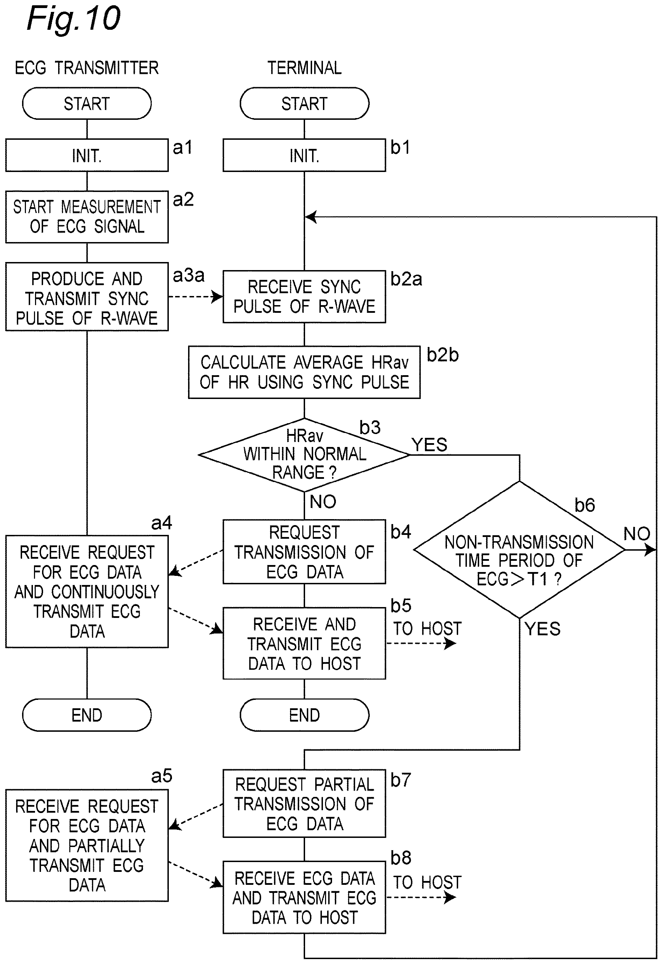

Fig. 10 is a flowchart showing further another example of the operations of the electrocardiographic transmitter 1 and the terminal 50. This example describes the case where the electrocardiographic transmitter 1 produces and wireless transmits a synchronous pulse SYN of the electrocardiogram waveform, and the terminal 50 calculates the average value HRav using this synchronous pulse SYN and determines normality or abnormality of the average value HRav. When the electrocardiographic transmitter 1 is attached to the chest of the subject PA and the power switch 11 is turned on, initialization is executed at step a1 to make initial settings for, for example, the detection threshold value for the R-wave. Meanwhile, when the dedicated software installed in the terminal 50 is started up, initialization is executed at step b1 to make initial settings for, for example, the interval to calculate the average value HRav of the heart rate HR, the upper limit and the lower limit of the normal range of the average value HRav of the heart rate HR, the threshold value of the non-transmission time period of the electrocardiogram data, and the data amount of the partial transmission of the electrocardiogram data. Out of these initially set values, at least the upper limit and the lower limit of the normal range of the average value HRav of the heart rate HR and the threshold value of the non-transmission time period of the electrocardiogram data may optionally be set by a person such as the subject PA or the medical worker.

The electrocardiographic transmitter 1 next starts measurement of the electrocardiographic signal at step a2, and produces and transmits the synchronous pulse SYN of the R-wave to the terminal 50 at step a3a.

The terminal 50 receives the synchronous pulse SYN transmitted from the electrocardiographic transmitter 1 at step b2a, and then calculates the average value HRav of the heart rate HR using the synchronous pulse SYN at step b2b, and then determines whether or not the average value HRav is within the normal range (such as, 30 to 160 bpm) at step b3. When the average value HRav is within the normal range, the operation returns through step b6 (for more detail, see the description relating to steps b6 to b8 in Fig. 5) to step b2. In this manner, it can be determined that the subject PA is in a lull in the case where the average value HRav is normal. Thus, the electric power necessary for the data transmission can therefore be saved by omitting transmission of the electrocardiogram data having a huge data amount to the terminal 50.

On the other hand, in the case where the average value HRav is out of the normal range at step b3, it can be determined that the subject PA is in a serious condition, and the operation therefore moves to step b4 and the terminal 50 requests the electrocardiographic transmitter 1 to transmit the electrocardiogram data. The electrocardiographic transmitter 1 receives a request order for the electrocardiogram data and thereafter continuously transmits the electrocardiogram data based on the electrocardiographic signal to the terminal 50 at step a4. The terminal 50 receives the continuously transmitted electrocardiogram data and transfers the electrocardiogram data as it is to the host device 70 at step b5. In this manner, in the case where the average value HRav is abnormal, all of the electrocardiogram data of the subject PA can be supplied to the medical worker.

This example describes the case where the synchronous pulse SYN synchronizing with the R-wave is produced. Alternatively, a synchronous pulse SYN synchronizing with P-wave, Q-wave, S-wave, or T-wave of the electrocardiogram waveform shown in Fig. 9A can also be produced.

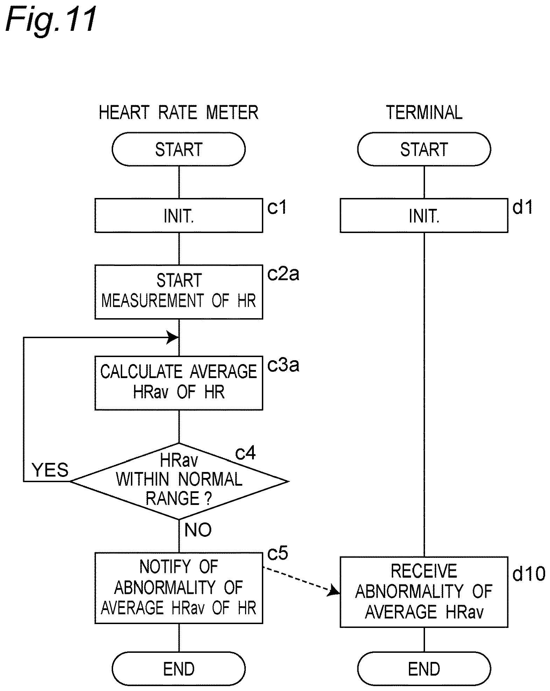

Next, a communication-type heart rate meter to which the electrocardiographic transmitter 1 is applied will be described below. The heart rate meter has a function of measuring the heart rate by optically detecting a variation of the blood flow in a blood vessel, instead of the electrocardiographic signal, and can be attached to, for example, chest, wrist, arm, or neck. Fig. 11 is a flowchart showing one example of operations of the heart rate meter and the terminal. In this flowchart, steps c6, c7, and d2 to d7 in the flowchart shown in Fig. 6 are omitted, and step c2a is executed instead of step c2, for starting the measurement of the heart rate, and step c3a is executed instead of step c3, for calculating the average value HRav of the heart rate HR in a predetermined period of time (such as, 1 minute) without data transmission. The heart rate meter executes threshold determination for the average value HRav at subsequent step c4, while the heart rate meter transmits nothing to the terminal 50 as long as the average value HRav is normal, however, in the case where the average value HRav is abnormal, the heart rate meter notifies the terminal 50 of the abnormality (step c5) and the terminal 50 receives the notification of the abnormality of the average value HRav (step d10). The frequency of the data communication is thereby further reduced, and reduction of the power consumption and extension of the battery life can therefore be facilitated.

The above-described embodiments exemplify an approach of monitoring the state of the subject PA in a simplified manner using the average value HRav of the heart rate HR as the amount of statistics of the electrocardiographic signal. For such amount of statistics, at least one parameter selected from the group consisting of the average value, the minimal value, the maximal value, the median value, the most frequent value, the dispersion, and the standard deviation of the heart rate in a predetermined period of time may be used. In this regard the average value means a value acquired by dividing the total value of the data by the number of data. The minimal value means the smallest value among the data. The maximal value means the greatest value among the data. The median value means a value positioned at the center when the data are rearranged in ascending order and, or, if the number of data is even, the average of the two values close to the center. The most frequent value means a value of the data whose frequency is the largest. The dispersion means an average value of the squares of differences each between the average value and the individual data. The standard deviation means a positive square root of the dispersion.

### [INDUSTRIAL APPLICABILITY]

The present invention is industrially very useful since a state of the heart of a subject can be monitored for a long term.

### [EXPLANATORY NOTE]

- 1: ELECTROCARDIOGRAPHIC (ECG) TRANSMITTER
- 2, 3, 4: ELECTRODE PAD
- 10: MAIN BODY
- 11: POWER SWITCH
- 12, 13, 14: TERMINAL
- 12a, 13a, 14a: FASTENER
- 15: BATTERY COVER
- 21: DIFFERENTIAL AMPLIFIER
- 22: FILTER
- 23: AMPLIFIER
- 24: R-WAVE DETECTION CIRCUIT
- 25: VIBRATION SENSOR
- 26: RADIO COMMUNICATION CIRCUIT
- 27: ANTENNA
- 30: CPU
- 31: A/D CONVERTER
- 32: MEMORY
- 33: POWER SOURCE CIRCUIT
- 50: TERMINAL
- 51: DISPLAY
- 70: HOST DEVICE
- PA: SUBJECT
- SYN: SYNCHRONOUS PULSE

## Claims

1. An electrocardiographic measurement system comprising:
an attachable electrocardiographic transmitter that includes an electrocardiographic signal detection circuit configured to detect an electrocardiographic signal and a wireless communication circuit configured to wireless transmit the electrocardiographic signal, the transmitter capable of being attached to the body of a subject; and
a terminal configured to receive the electrocardiographic signal that is wireless transmitted from the electrocardiographic transmitter;
wherein the electrocardiographic transmitter executes a statistical process for the electrocardiographic signal to compute an amount of statistics and then transmits the amount of statistics to the terminal, and
the terminal determines whether or not the received amount of statistics is out of a predetermined range, and
the electrocardiographic transmitter transmits electrocardiogram data based on the electrocardiographic signal to the terminal in one case where the terminal determines that the amount of statistics is out of the predetermined range, and, on the other hand, does not transmit the electrocardiogram data to the terminal in the other case where the terminal determines that the amount of statistics is within the predetermined range.

2. An electrocardiographic measurement system comprising:
an attachable electrocardiographic transmitter that includes an electrocardiographic signal detection circuit configured to detect an electrocardiographic signal and a wireless communication circuit configured to wireless transmit the electrocardiographic signal, the transmitter capable of being attached to the body of a subject; and
a terminal configured to receive the electrocardiographic signal that is wireless transmitted from the electrocardiographic transmitter;
wherein the electrocardiographic transmitter executes a statistical process for the electrocardiographic signal to compute an amount of statistics, and then determines whether or not the amount of statistics is out of a predetermined range and transmits electrocardiogram data based on the electrocardiographic signal to the terminal in one case where the electrocardiographic transmitter determines that the amount of statistics is out of the predetermined range, and, on the other hand, does not transmit the electrocardiogram data to the terminal in the other case where the electrocardiographic transmitter determines that the amount of statistics is within the predetermined range.

3. An electrocardiographic measurement system comprising:
an attachable electrocardiographic transmitter that includes an electrocardiographic signal detection circuit configured to detect an electrocardiographic signal and a wireless communication circuit configured to wireless transmit the electrocardiographic signal, the transmitter capable of being attached to the body of a subject; and
a terminal configured to receive the electrocardiographic signal that is wireless transmitted from the electrocardiographic transmitter;
wherein the electrocardiographic transmitter produces a synchronous signal synchronizing with the electrocardiographic signal and transmits the synchronous signal to the terminal, and
the terminal computes an amount of statistics of the electrocardiographic signal from the received amount of statistics and determines whether or not the amount of statistics is out of a predetermined range, and
the electrocardiographic transmitter transmits electrocardiogram data based on the electrocardiographic signal to the terminal in one case where the terminal determines that the amount of statistics is out of the predetermined range, and, on the other hand, does not transmit the electrocardiogram data to the terminal in the other case where the terminal determines that the amount of statistics is within the predetermined range.

4. The electrocardiographic measurement system according to any one of Claims 1 to 3, further comprising a host device configured to receive and store therein the electrocardiogram data wireless transmitted from the terminal.

5. The electrocardiographic measurement system according to any one of Claims 1 to 3, wherein the amount of statistics is at least one parameter selected from the group consisting of average value, minimal value, maximal value, median value, most frequent value, dispersion, and standard deviation of a heart rate in a predetermined period of time.

6. The electrocardiographic measurement system according to any one of Claims 1 to 3, wherein the electrocardiographic transmitter transmits a portion of the electrocardiogram data to the terminal at a predetermined time interval even in the case where the amount of statistics is within the predetermined range.

7. An attachable electrocardiographic transmitter comprising:
an electrocardiographic signal detection circuit configured to detect an electrocardiographic signal; and
a wireless communication circuit configured to wireless transmit the electrocardiographic signal to an external terminal;
the transmitter capable of being attached to the body of a subject,
wherein the electrocardiographic transmitter execute a statistical process for the electrocardiographic signal to compute a amount of statistics and then transmits the amount of statistics to the terminal, and transmits electrocardiogram data based on the electrocardiographic signal to the terminal in one case where the amount of statistics is out of a predetermined range, and, on the other hand, does not transmit the electrocardiogram data to the terminal in the other case where the amount of statistics is within the predetermined range.

8. An attachable electrocardiographic transmitter comprising:
an electrocardiographic signal detection circuit configured to detect an electrocardiographic signal; and
a wireless communication circuit configured to wireless transmit the electrocardiographic signal to an external terminal;
the transmitter capable of being attached to the body of a subject,
wherein the electrocardiographic transmitter executes a statistical process for the electrocardiographic signal to compute a amount of statistics, and determines whether or not the amount of statistics is out of a predetermined range, and then transmits electrocardiogram data based on the electrocardiographic signal to the terminal in one case where the amount of statistics is out of a predetermined range, and, on the other hand, does not transmit the electrocardiogram data to the terminal in the other case where the amount of statistics is within the predetermined range.

9. An attachable electrocardiographic transmitter comprising:
an electrocardiographic signal detection circuit configured to detect an electrocardiographic signal and
a wireless communication circuit configured to wireless transmit the electrocardiographic signal to an external terminal;
the transmitter capable of being attached to the body of a subject,
wherein the electrocardiographic transmitter produces a synchronous signal synchronizing with the electrocardiographic signal and transmits the synchronous signal to the terminal, and transmits electrocardiogram data based on the electrocardiographic signal to the terminal in one case where a amount of statistics of the electrocardiographic signal computed by the terminal from the synchronous signal is out of a predetermined range, and, on the other hand, does not transmit the electrocardiogram data to the terminal in the other case where the amount of statistics is within the predetermined range.

10. The electrocardiographic transmitter according to any one of Claims 7 to 9, wherein the amount of statistics is at least one parameter selected from the group consisting of average value, minimal value, maximal value, median value, most frequent value, dispersion, and standard deviation of a heart rate in a predetermined period of time.
